# EUROPEAN PATENT APPLICATION

(11) **EP 4 506 634 A1**
(43) Date of publication of application: **12.02.2025**
(21) Application number: 23784604.3
(22) Date of filing: 15.03.2023
(51) Int. Cl.: F24F 8/22, A61L 9/20, F24F 1/0076, F24F 3/16, F24F 7/003

(54) **STERILIZATION UNIT**

(30) Priority: 05.04.2022 JP 2022062966
(71) Applicant: Carrier Japan Corporation, Tokyo 141-0032 (JP)
(72) Inventor: OZAWA, Tetsuro, Fuji-shi, Shizuoka 416-8521 (JP)
(74) Representative: Gramm, Lins & Partner Patent- und Rechtsanwälte PartGmbB
(86) International application number: PCT/JP2023/010009
(87) International publication number: WO 2023/195312

(57) **Abstract**

To Provide a sterilization unit (11) whereby inspection and a replacement operation of an ultraviolet lamp (32) can be carried out safely. A sterilization unit (11) is provided with: a casing (12), maintenance panels (41, 51) that are removably attached to the outer surface of the casing (12), an ultraviolet lamp (32) that sterilizes air passing through the interior of the casing (12), a control board (65a) for the ultraviolet lamp (32), and an electrical component box (61) in which the control board (65a) is accommodated. The casing (12) is provided with a shield unit (101) which prevents ultraviolet light emitted by the ultraviolet lamp (32) from leaking to the exterior of the casing (12) through screw holes for attaching the maintenance panels (41, 51).

## Description

### Technical Field

An embodiment of the present invention relates to a sterilization unit.

### Background Art

The impact of novel coronavirus (COVID-19) infections has increased the need for improved air quality environments as a measure to prevent infection.

For example, Patent Document 1 discloses an air conditioning system including an ultraviolet lamp in an indoor unit or in an air circulation passage of the air conditioning system to eliminate germs contained in the air.

### Prior Art Documents

### Patent Document

Patent Document 1: JP 2022-042589 A

### Summary of the Invention

### Problems to be Solved by the Invention

Ultraviolet light is categorized into UV-A, UV-B, and UV-C. Among these categories, UV-C is said to have a significant effect on germs. On the other hand, UV-C may have adverse effects on the human body when the human body is directly exposed to UV-C. During inspection or replacement of ultraviolet lamps that generate UV-C, it is necessary to ensure that the ultraviolet lamps that generate UV-C are turned off.

An object of the present invention is to provide a sterilization unit that allows inspecting work or replacing work of the ultraviolet lamps to be performed safely.

### Means for Solving Problem

To achieve the above object, an aspect of the present invention provides a sterilization unit including: a casing, an ultraviolet lamp that sterilizes air passing through the casing, a control board for the ultraviolet lamp, and an electric component box that houses the control board. The casing includes an internal casing provided inside the casing, and a shielding portion that is part of the internal casing to prevent ultraviolet light emitted by the ultraviolet lamp from leaking to outside of the casing.

It may be further desired that a maintenance panel that is detachably attached to an outer surface of the casing. The shielding portion prevents the ultraviolet light from leaking to the outside of the casing through screw holes for attaching the maintenance panel.

Furthe, to achieve the above object, an aspect of the present invention provides a sterilization unit including: a casing, a maintenance panel that is detachably attached to an outer surface of the casing, an ultraviolet lamp that sterilizes air passing through the casing, a control board for the ultraviolet lamp, and an electric component box that houses the control board. The casing includes a shielding portion that prevents ultraviolet light emitted by the ultraviolet lamp from leaking to outside of the casing through screw holes for attaching the maintenance panel.

It may be desired that the shielding portion is part of an internal casing provided inside the casing.

It may be desired that the internal casing covers part of a cable cover that houses a cable connected to the ultraviolet lamp.

### Effects of Invention

The present invention can provide a sterilization unit that allows inspecting work or replacing work of the ultraviolet lamps to be performed safely.

### Brief Description of the Drawings

Fig. 1 is a side view schematically showing an indoor unit and a sterilization unit of an air conditioner of an embodiment being installed above a ceiling.
Fig. 2 is a perspective view of the sterilization unit according to the embodiment.
Fig. 3 is a plan view of the sterilization unit according to the embodiment as viewed from the side of a blowout port of the sterilization unit.
Fig. 4 is a sectional view of the sterilization unit in Fig. 3 along an X-X line.
Fig. 5 is an exploded perspective view of a shading grille.
Fig. 6 is a sectional view of the shading grille.
Fig. 7 is a schematic perspective view of an ultraviolet lamp unit.
Fig. 8 is a perspective view showing maintenance panels being removed.
Fig. 9 is a plan view of a first maintenance panel.
Fig. 10 is a plan view of a second maintenance panel.
Fig. 11 is a side view of the sterilization unit with the first maintenance panel and the second maintenance panel being removed as viewed from the side of the first side plate.
Fig. 12 is a perspective view showing the state where a first lid part is opened.
Fig. 13 is a perspective view showing the state where a second lid part is opened.
Fig. 14 is a perspective view showing the state where the second maintenance panel is removed in the state of Fig. 13.
Fig. 15 is a schematic diagram showing the detection mechanism.
Fig. 16 is a perspective view of the first side plate as viewed from the inside of a casing.
Fig. 17 is a perspective view of an internal casing.
Fig. 18 is a perspective view of a cable cover.
Fig. 19 is an enlarged view of an R portion of Fig. 4.

### Description of Embodiments

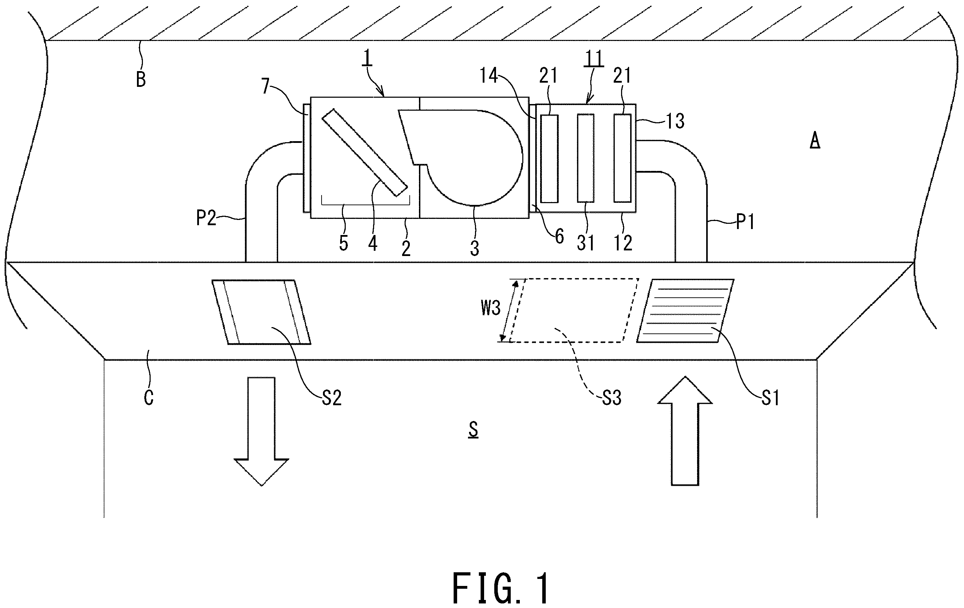

An embodiment of a sterilization unit according to the present invention is described with reference to Figs. 1 to 19. In the drawings, identical or corresponding component members are designated by identical reference signs.

Fig. 1 is a side view schematically showing an indoor unit and the sterilization unit of an air conditioner installed above a ceiling.

As shown in Fig. 1, the indoor unit 1 of the air conditioner (hereinafter simply referred to as "air conditioning indoor unit 1") and a sterilization unit 11 are installed, for example, above a ceiling of a building. In the present embodiment, the term "above a ceiling" refers to a ceiling space A defined between a beam B and a ceiling panel C of the building.

The air conditioning indoor unit 1 is a duct-type air conditioning indoor unit for a ceiling-embedded air conditioner. The air conditioning indoor unit 1 includes a casing 2, a blower 3, a heat exchanger 4, and a drain pan 5. The blower 3, the heat exchanger 4, and the drain pan 5 are provided inside the casing 2. The casing 2 includes a suction port 6 and a blowout port 7.

The sterilization unit 11 according to the present embodiment, which is connected to the indoor unit of the ceiling-embedded air conditioner, emits ultraviolet light (UV-C) from an ultraviolet lamp 32 to eliminate germs contained in the air in the building. The sterilization unit 11 is coupled to the suction port 6 of the air conditioning indoor unit 1. The sterilization unit 11 includes a casing 12, and at least one ultraviolet lamp unit 31 provided inside the casing 12. The casing 12 includes the suction port 13 and the blowout port 14.

The ceiling panel C has an indoor air suction port S1 that draws in indoor air that is the air in an air conditioning target space S, an air-conditioned air blowout port S2 that blows out air-conditioned air, passing through the sterilization unit 11 and the air conditioning indoor unit 1, to the air conditioning target space S, and a maintenance hatch S3. The indoor air suction port S1 and the suction port 13 of the sterilization unit 11 are connected through a suction duct P1, and the air-conditioned air blowout port S2 and the blowout port 7 of the air conditioning indoor unit 1 are connected through a blowout duct P2.

Indoor air is drawn through the indoor air suction port S1 and flows into the sterilization unit 11 via the suction duct P1 by rotating driven the blower 3. The indoor air flowing into the sterilization unit is sterilized by the ultraviolet lamp unit 31. The sterilized indoor air flows from the sterilization unit 11 into the air conditioning indoor unit 1 and exchanges heat with a refrigerant flowing inside the heat exchanger 4. The indoor air heat-exchanged in the heat exchanger 4 is blown out as cold or warm air from the air-conditioned air blowout port S2 to the air conditioning target space S through the blowout duct P2.

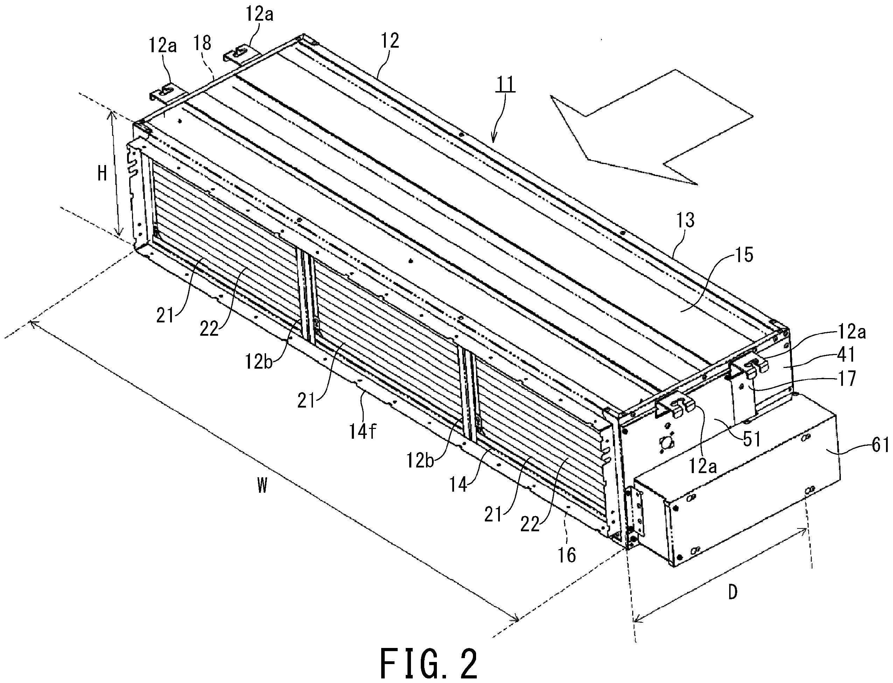

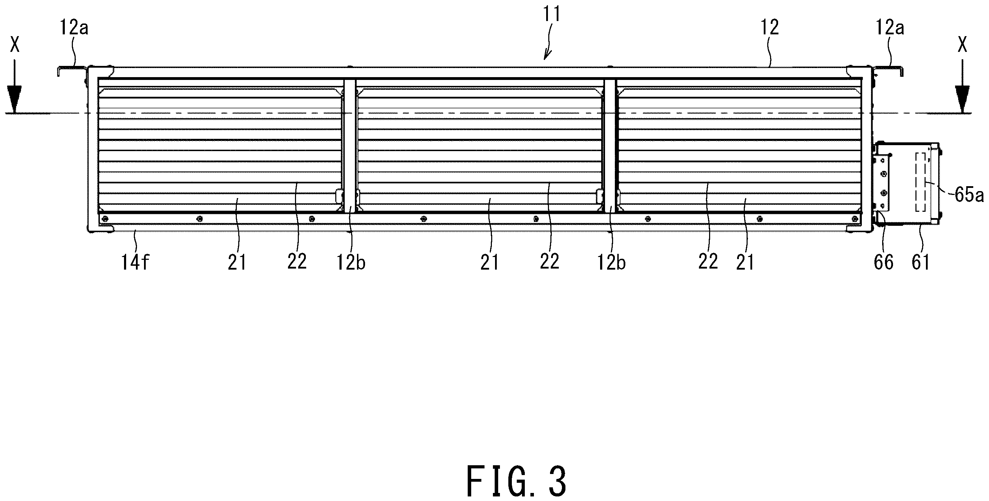

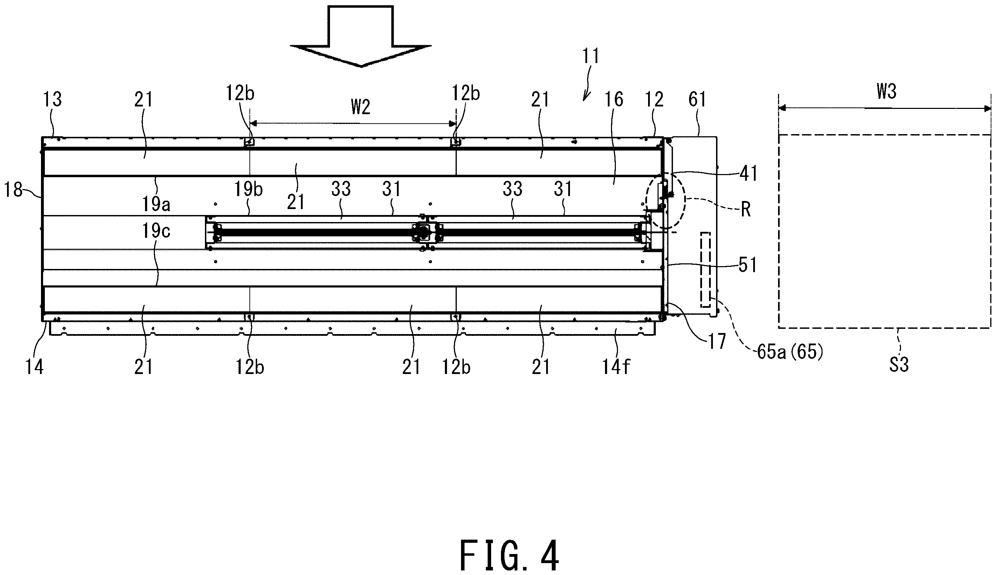

The sterilization unit 11 is described with reference to Figs. 2 to 4. Fig. 2 is a perspective view of the sterilization unit, Fig. 3 is a plan view of the sterilization unit as viewed from the blowout port side, and Fig. 4 is a sectional view of the sterilization unit taken along an X-X line in Fig. 3.

As shown in Figs. 2 to 4, the sterilization unit 11 according to the present embodiment includes the casing 12, and an electric component box 61 that houses an electric component 65 including a control board 65a of the ultraviolet lamp unit 31.

The casing 12 of the sterilization unit 11 has the shape of a flat square box, with a depth dimension D, a width dimension W, and a thickness dimension H. The depth dimension D of the casing 12 is smaller than the width dimension W of the casing 12. The thickness dimension H of the casing 12 is smaller than the depth dimension D and the width dimension W of the casing 12.

The casing 12 is a box body made of a metal plate, such as a sheet metal, for example. The casing 12 includes, as main elements, a top plate 15, a bottom plate 16, a first side plate 17, and a second side plate 18.

The top plate 15 and the bottom plate 16 extend horizontally. The top plate 15 and the bottom plate 16 face each other at a distance in the thickness direction of the casing 12. The first side plate 17 and the second side plate 18 stand so as to straddle over the top plate 15 and the bottom plate 16. The first side plate 17 and the second side plate 18 face each other at a distance in the width direction of the casing 12.

The casing 12 includes a plurality of hanging metal fittings 12a provided at each of an upper portion of the first side plate 17 and an upper portion of the second side plate 18. The hanging metal fittings 12a are coupled to hanging bolts (not shown) extending from the beam B of the building shown in Fig. 1 so that the sterilization unit 11 is hung in the ceiling space A.

A pair of side surfaces facing each other in the depth direction of the casing 12 is widely opened in the width direction of the casing 12. One opening portion serves as the suction port 13, while the other opening portion serves as the blowout port 14. The suction port 13 is connected to the suction duct P1, and the blowout port 14 is connected to the suction port 6 of the air conditioning indoor unit 1 via a connection flange 14f.

The casing 12 houses at least one shading grille 21 facing the suction port 13, at least one shading grille 21 facing the blowout port 14, and the ultraviolet lamp unit 31 disposed between the shading grille 21 facing the suction port 13 and the shading grille 21 facing the blowout port 14. In the present embodiment, the distance between the shading grille 21 facing the suction port 13 and the shading grille 21 facing the blowout port 14 is set to 200 millimeters (mm) or more, and more specifically about 300 millimeters (mm). The distance between the shading grills 21 ensures a sufficient space for sterilization.

In the present embodiment, the sterilization unit 11 includes three shading grilles 21 facing the suction port 13 and three shading grilles 21 facing the blowout port 14. The three shading grilles 21 facing the suction port 13 are aligned in the width direction of the casing 12. The three shading grilles 21 facing the blowout port 14 are aligned in the width direction of the casing 12. Here, the number of the shading grills 21 aligned in the width direction of the casing 12 is not limited to three, and can be increased or decreased according to the size of the casing 12 and the width dimension of the shading grills 21.

In the present embodiment, the sterilization unit 11 includes two ultraviolet lamp units 31 aligned in the width direction of the casing 12. Here, the number of the ultraviolet lamp units 31 aligned in the width direction of the casing 12 is not limited to two, and can be increased or decreased according to the size of the casing 12 and the width dimension of the ultraviolet lamp units 31.

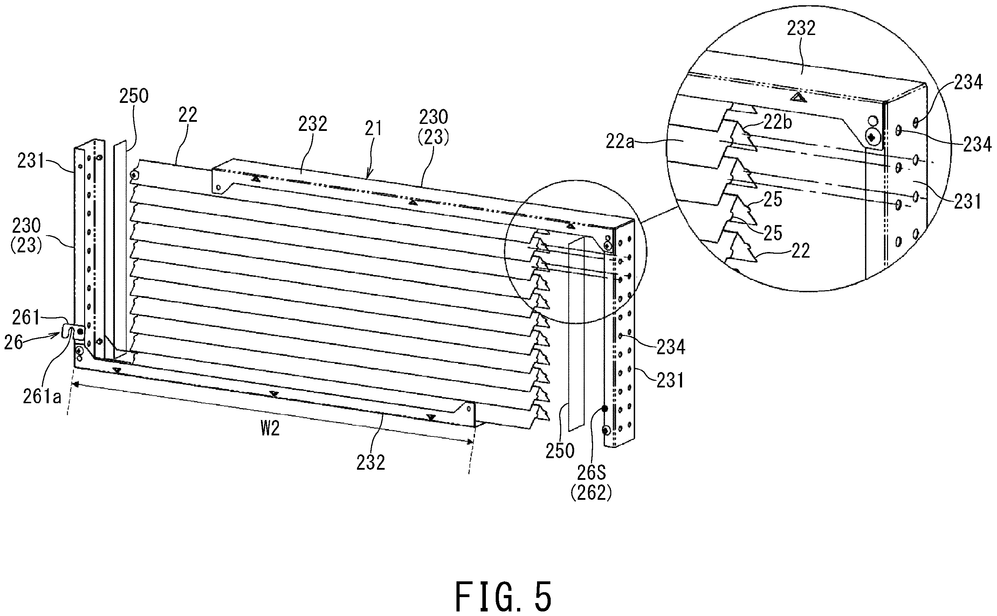

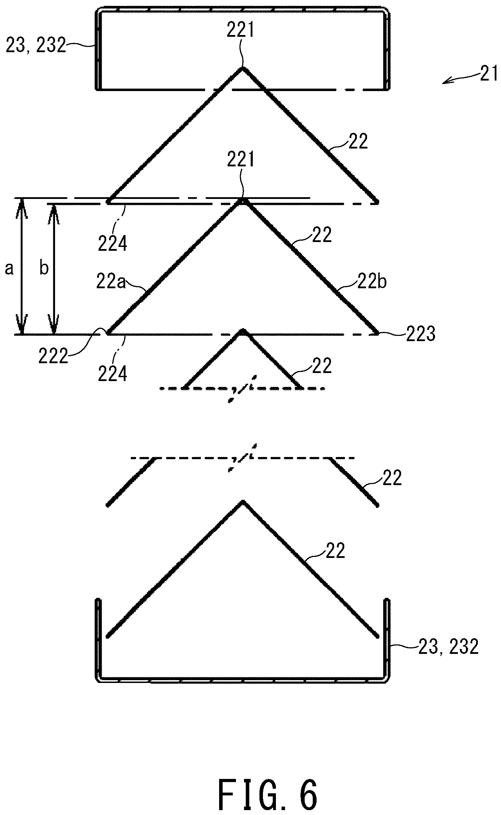

The shading grilles 21 are described with reference to Figs. 5 and 6. Fig. 5 is an exploded perspective view of the shading grille 21, and Fig. 6 is a sectional view of the shading grille.

As shown in Figs. 5 and 6, the shading grille 21 of the sterilization unit 11 according to the present embodiment includes a plurality of inverted V-shaped louvers 22 that overlap at specified intervals, and a rectangular frame body 23 enclosing the plurality of louvers 22.

The frame body 23 includes two L-shaped members 230. The L-shaped members 230 each have a longitudinal member 231 corresponding to the short side of the shading grille 21, and a transverse member 232 corresponding to the long side of the shading grille 21. The L-shaped members 230 have a U-shaped cross section. The rectangular frame body 23 is formed by engaging both end portions of the two L-shaped members with fastening members such as screws.

A length dimension W2 of the transverse members 232 of the frame body 23, which is a longitudinal length dimension of the shading grille 21, is set to be smaller than a width dimension W3 of the maintenance hatch S3 shown by dotted lines in Figs. 1 and 4.

The louvers 22 are molded articles each made of a thin metal sheet, such as a sheet metal. The cross-sectional shape of each of the louvers 22 is an inverted V-shaped with an approximately 90° bend. The cross-sectional shape of each of the louvers 22 is line-symmetric with respect to an apex 221. The apex 221, one lower end 222, and the other lower end 223 are connected to form a rectangular equilateral triangle. The louvers 22 each have a first plate portion 22a extending between the apex 221 and one lower end 222 and a second plate portion 22b extending between the apex 221 and the other lower end 223.

One louver 22 is disposed so as to overlap with another louver 22. The plurality of louvers 22 are disposed so that a distance "a" from a virtual line 224, which connects one lower end 222 and the other lower end 223 of each of the louvers 22, to the apex 221 of each of the louvers 22 is larger than a distance "b" that is between the virtual lines 224 of the adjacent louvers 22. This ensures that the ultraviolet light coming from the ultraviolet lamp unit 31 does not leak to the outside of the shading grille 21. It also becomes possible to prevent the leakage of ultraviolet light and to reduce loss of airflow pressure by setting the distance "a" to be larger than the distance "b" and also minimizing the difference between the distance "a" and the distance "b".

The louvers 22 each include a plurality of protrusions 25 at both end portions in the longitudinal direction. The protrusions 25 further extend from the end portions of the louver 22 in the longitudinal direction of the louver 22. One protrusion 25 is provided at each of both end portions of the first plate portion 22a, and one protrusion 25 is provided at each of both end portions of the second plate portion 22b. The protrusions 25 provided in the first plate portion 22a and the protrusions 25 provided in the second plate portion 22b are equal in distance from the apex 221.

The frame body 23 includes a plurality of engagement portions 234 corresponding to the protrusions 25. Each of the engagement portions 234 is approximately a circular hole. The plurality of engagement portions 234 are provided in the longitudinal members 231 of the frame body 23. Specifically, the plurality of engagement portions 234 are arranged in two rows in the longitudinal direction of the longitudinal members 231. In a direction orthogonal to the longitudinal direction of the longitudinal member 231, two engagement portions 234 are adjacent to each other so as to correspond to the two protrusions 25 at one end of one louver 22. In the longitudinal direction of the longitudinal member 231, the plurality of engagement portions 234 are aligned in accordance with the number of the louvers 22 so that the adjacent louvers 22 are disposed at intervals of the distance "b". When the protrusions 25 of each of the louvers 22 are inserted into the engagement portions 234, the louvers 22 are fixed to the frame body 23. With this assembly structure of the protrusions 25 and the engagement portions 234, the plurality of louvers 22 are easily retained within the frame body 23 at intervals of the constant distance "b".

Seal members 250 are provided between the louvers 22 and the longitudinal members 231 of the frame body 23. Each of the seal members 250 is disposed between two rows of the engagement portions 234. The seal members 250 reduce the noise produced by the contact between the louvers 22 and the frame body 23.

The shading grilles 21 each include a coupling mechanism 26 that couples the adjacent shading grilles 21. The coupling mechanism 26 has a coupling hook portion 261 provided in the frame body 23, and a coupling projection portion 262 provided in the frame body 23. The coupling hook portion 261 is provided in one longitudinal member 231 of the frame body 23, and the coupling projection portion 262 is provided in the other longitudinal member 231 of the frame body 23. The coupling hook portion 261 has a hook groove 261a that can hook the coupling projection portion 262. The hook groove 261a of the coupling hook portion 261 is designed to be caught by the coupling projection portion 262 of the adjacent shading grille 21. In the present embodiment, the coupling projection portion 262 is a shaft part of a screw 26S fixed to the longitudinal member 231. With the thus-configured coupling mechanism 26 in the sterilization unit 11 of the present embodiment, three shading grilles 21 are coupled in the width direction of the sterilization unit 11 as shown in Fig. 2.

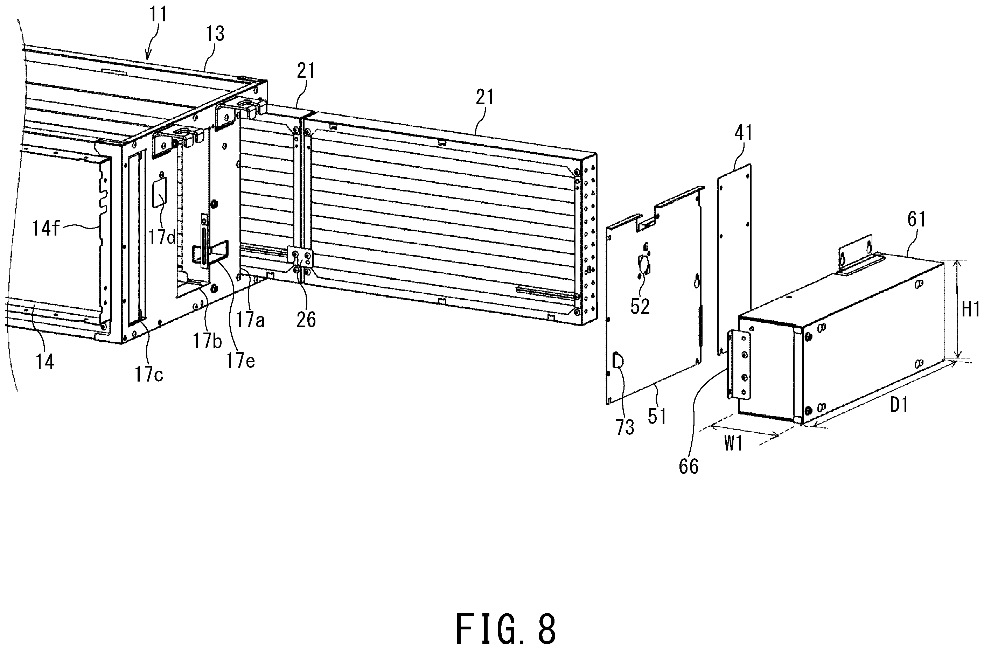

For maintenance and cleaning of the shading grilles 21, the shading grilles 21 can be removed one by one from the sterilization unit 11 by canceling the coupling of the coupling mechanism 26. Specifically, as shown in Fig. 8, coupling of the coupling mechanism 26 is canceled while at least one shading grille 21 is fully pulled out of the casing 12 of the sterilization unit 11. Since the dimension W2 in the longitudinal direction of the shading grilles 21 is set to be smaller than the width dimension W3 of the maintenance hatch S3, the shading grilles 21 can easily be moved in and out of the maintenance hatch S3, which improves workability.

When the plurality of shading grilles 21 are coupled and installed, small gaps may be generated between the adjacent shading grilles 21. Ultraviolet light may leak to the outside of the sterilization unit 11 through these gaps. Accordingly, as shown in Fig. 2, the casing 12 of the sterilization unit 11 includes columns 12b to seal the gaps between the coupling points of the shading grilles 21. The columns 12b can reliably prevent the leakage of ultraviolet light to the outside of the casing 12 without the need for complex structure.

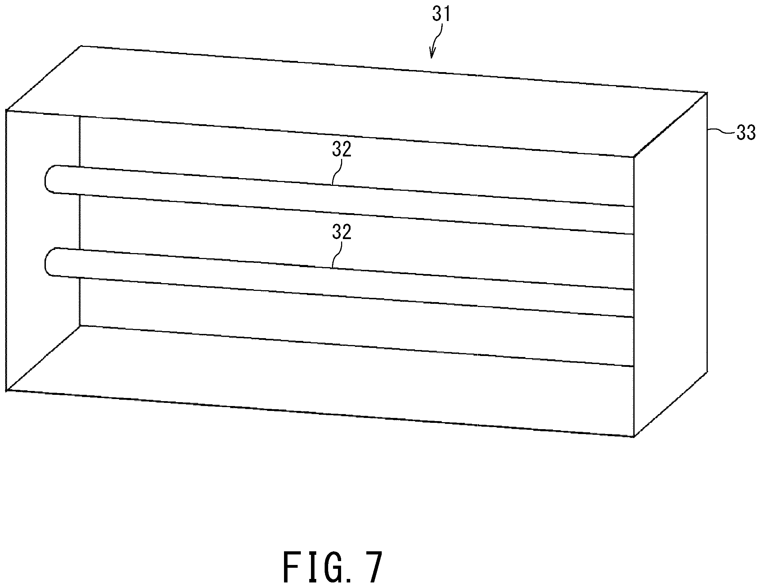

The ultraviolet lamp units 31 housed in the sterilization unit 11 is described using Fig. 7. Fig. 7 is a perspective view of the ultraviolet lamp unit 31.

As shown in Fig. 7, the ultraviolet lamp unit 31 of the sterilization unit 11 according to the present embodiment includes the ultraviolet lamps 32 and a frame body 33 that fixes the ultraviolet lamps 32. The ultraviolet lamps 32 emit ultraviolet light of 100 nanometers (nm) to 280 nanometers (nm), which is classified as UV-C. The plurality of ultraviolet lamps 32, that is, two ultraviolet lamps 32, for example, are attached to the frame body so as to be aligned at upper and lower sides in the thickness (height) direction of the sterilization unit 11 and to extend horizontally in the width direction of the sterilization unit 11.

The frame body 33 of the ultraviolet lamp unit 31 includes a coupling mechanism (not shown). When two ultraviolet lamp units 31 are housed in the casing 12, the adjacent ultraviolet lamp units 31 are electrically connected. It is desirable that the length dimension of the ultraviolet lamp units 31 in the longitudinal direction is set to be smaller than the width dimension W3 of the maintenance hatch S3, as in the case of the shading grilles 21.

The casing 12 includes a first maintenance panel 41 and a second maintenance panel 51 for attaching and detaching the shading grilles 21 or the ultraviolet lamp units 31 to and from the sterilization unit 11. The first maintenance panel 41 and the second maintenance panel 51 are attached to the first side plate 17 of the casing 12.

The first maintenance panel 41 and the second maintenance panel 51 are described using Figs. 2, 4, 8, and 9 to 11. Fig. 8 is a perspective view showing the first maintenance panel 41 and the second maintenance panel 51 being removed from the sterilization unit 11. The first maintenance panel 41 and the second maintenance panel 51 are described using Figs. 2, 4 and 8.

As shown in Fig. 8, the first side plate 17 of the casing 12 of the sterilization unit 11 according to the present embodiment has an access hole 17a for the shading grilles 21 on the side of the suction port 13, an access hole 17b for the ultraviolet lamp units 31, and an access hole 17c for the shading grilles 21 on the side of the blowout port 14.

The first side plate 17 of the casing 12 further has an inspection hole 17d disposed between the access hole 17b for the ultraviolet lamp units 31 and the access hole 17c for the shading grilles 21 on the side of the blowout port 14.

As shown in Fig. 4, the casing 12 includes guide rails 19a, 19b, and 19c that guide the shading grilles 21 and the ultraviolet lamp units 31 to prescribed positions. The guide rails 19a, 19b, and 19c correspond to the access holes 17a, 17b, and 17c on the first side plate 17, respectively. The guide rails 19a, 19b, and 19c guide the shading grilles 21 and the ultraviolet lamp units 31 to prescribed positions inside the casing 12. The guide rails 19a, 19b, and 19c extend along the width of the casing 12 from the vicinity of the first side plate 17. Each of the guide rails 19a, 19b, and 19c is a pair of facing rails provided on the top plate 15 and the bottom plate 16 of the casing 12. The guide rails 19a and 19c correspond to the shading grilles 21, while the guide rail 19b corresponds to the ultraviolet lamp units 31.

As shown in Fig. 8, when the first maintenance panel 41 is removed, the access hole 17a for the shading grilles 21 on the side of the suction port 13 is exposed. When the second maintenance panel 51 is removed, the access hole 17b for the ultraviolet lamp units 31, the access hole 17c for the shading grilles 21 on the side of the blowout port 14, and the inspection hole 17d are exposed.

The second maintenance panel 51 includes an inspection window 52 that allows visual inspection of the inside of the sterilization unit 11 through the inspection hole 17d, and a piece portion 73 that is part of a detection mechanism 71 described later.

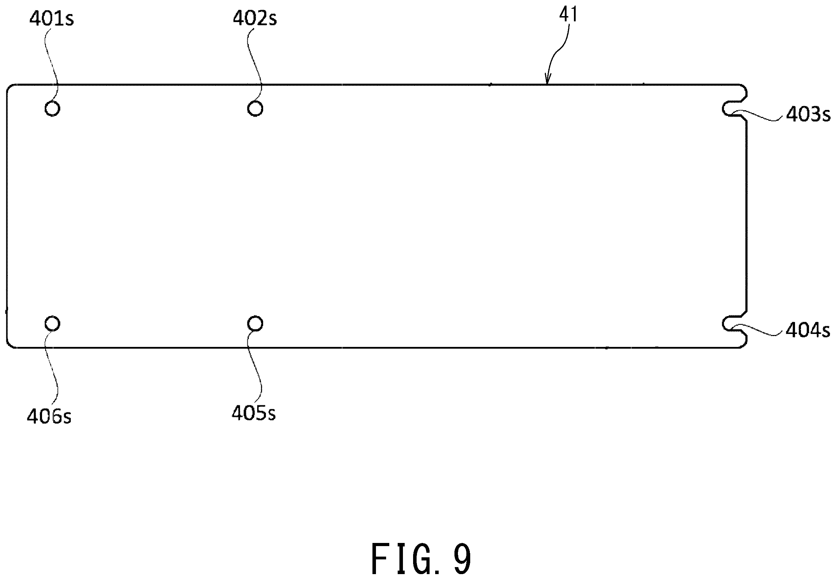

Fig. 9 is a plan view of the first maintenance panel 41. As shown in Fig. 9, the first maintenance panel 41 has a plurality of screw fixing holes 401s to 406s.

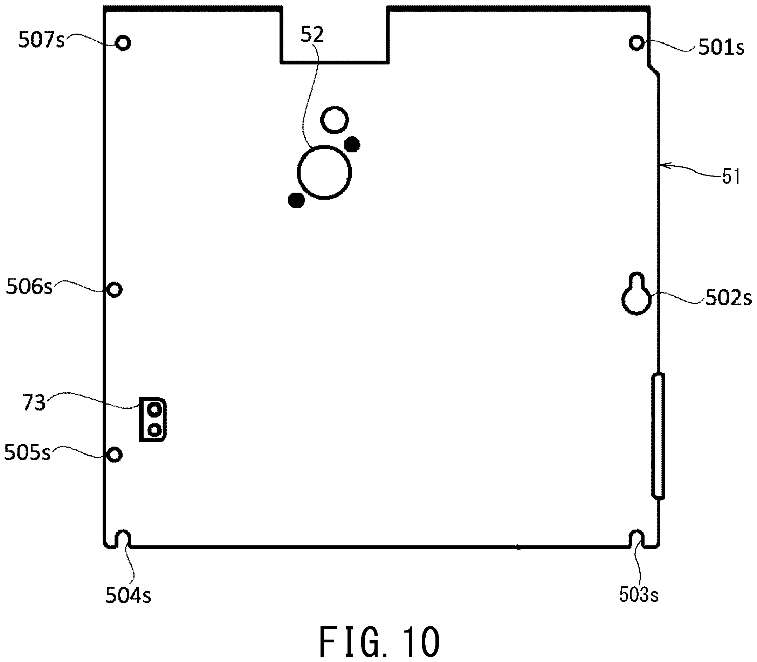

Fig. 10 is a plan view of the second maintenance panel 51. As shown in Fig. 10, the second maintenance panel 51 has a plurality of screw fixing holes 501s to 507s.

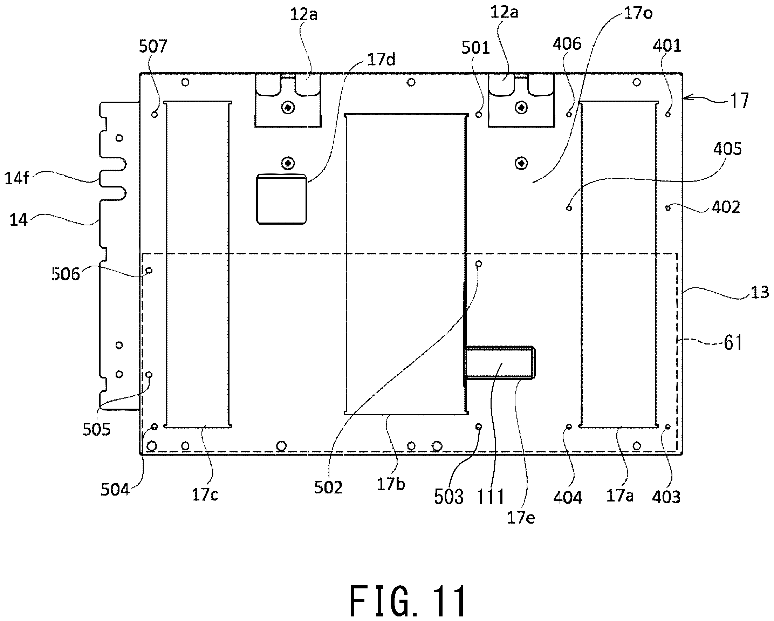

Fig. 11 is a side view of the sterilization unit 11 with the first maintenance panel 41 and the second maintenance panel 51 being removed as viewed from the side of the first side plate. As shown in Fig. 11, the first side plate 17 of the casing 12 has screw holes 401 to 406 corresponding to the screw fixing holes 401s to 406s of the first maintenance panel 41 and screw holes 501 to 507 corresponding to the screw fixing holes 501s to 507s of the second maintenance panel 51.

When panel fixing screws 17s are fastened into the screw fixing holes 401s to 406s, the screw fixing holes 501s to 507s, the screw holes 401 to 406, and the screw holes 501 to 507, the first maintenance panel 41 and the second maintenance panel 51 are threaded into the first side plate 17, and then the electric component box 61 is installed at a position indicated by a dotted line in Fig. 11.

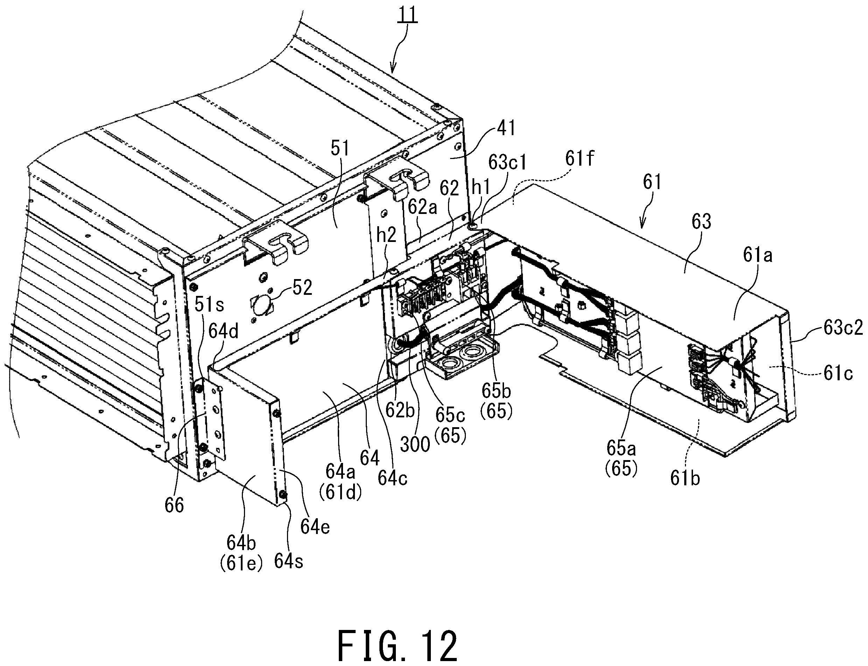

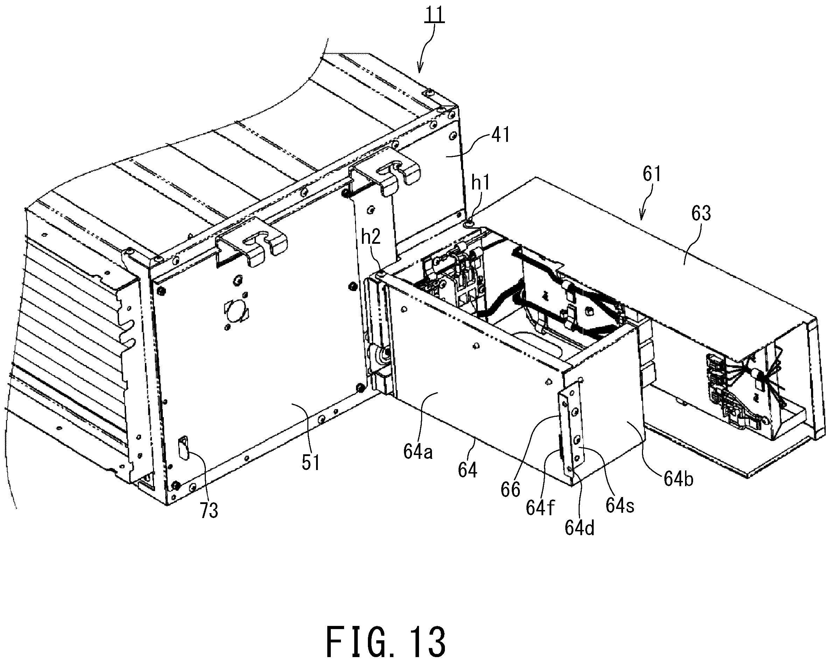

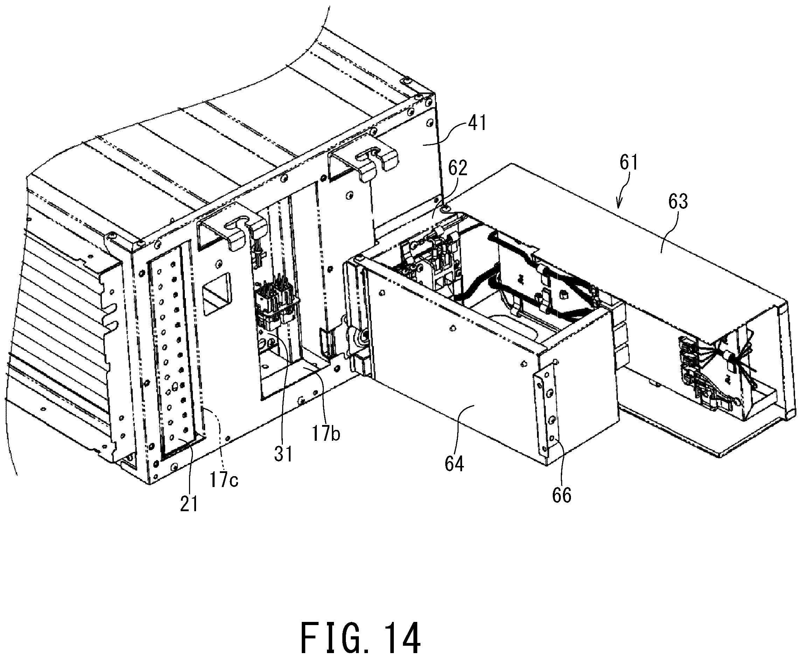

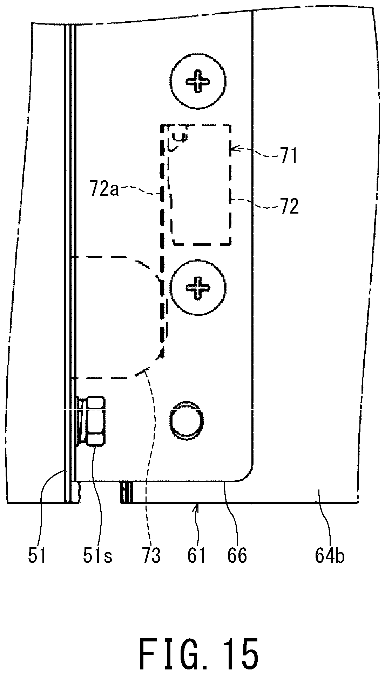

Fig. 12 is a perspective view showing the state where the first lid part is opened in the state of Fig. 2. Fig. 13 is a perspective view showing the state where the second lid part is opened in the state of Fig. 12. Fig. 14 is a perspective view showing the state where the second maintenance panel is removed in the state of Fig. 13. Fig. 15 is a schematic diagram showing the detection mechanism. Next, the electric component box 61 is described using Figs. 2 to 4 and 12 to 15.

As shown in Figs. 2 to 4, the electric component box 61 of the sterilization unit 11 according to the present embodiment is provided outside the first maintenance panel 41 and the second maintenance panel 51.

The electric component box 61 has a rectangular parallelepiped shape that is narrower and longer than the casing 12. The electric component box 61 has a depth dimension D1, a width dimension W1, and a thickness dimension H1. The depth dimension D1 of the electric component box 61 is substantially the same as the depth dimension D of the casing 12. The thickness dimension H1 of the electric component box 61 is about half the thickness dimension H of the casing 12. The width dimension W1 of the electric component box 61 is smaller than the width dimension W of the casing 12.

As shown in Figs. 12 to 14, the electric component box 61 includes a base part 62, a first lid part 63, and a second lid part 64. The base part 62, the first lid part 63, and the second lid part 64 are molded articles made of metal sheets, such as sheet metals. The base part 62, the first lid part 63, and the second lid part 64 define the rectangular parallelepiped outline of the electric component box 61. Specifically, the base part 62 defines an inner side surface portion 61d of the electric component box 61 together with the second lid part 64. The first lid part 63 defines an upper surface portion 61a, a lower surface portion 61b, an outer side surface portion 61c, and a rear surface portion 61f of the electric component box 61. The second lid part 64 defines a front surface portion 61e and part of the inner side surface portion 61d of the electric component box 61.

The base part 62 includes a fixed portion 62a that is fixed to the first maintenance panel 41 with a fastening member, such as a screw. In addition, the electric component 65, including a power terminal block 65b and an external control terminal block 65c that is connected to a communication line leading to the air conditioning indoor unit 1, is attached to the base part 62 inside the electric component box 61.

The base part 62 includes a first hinge portion h1 and a second hinge portion h2. The first hinge portion h1 swingably couples the first lid part 63 to the base part 62. The second hinge portion h2 swingably couples the second lid part 64 to the base part 62.

The first lid part 63 has a first corner portion 63c1 of the casing 12 of the sterilization unit 11 on the side of the suction port 13, and a second corner portion 63c2 that is positioned on a diagonal line of the first corner portion 63c1. The first corner portion 63c1 is rotatably attached to the first hinge portion h1 of the base part 62. The second corner portion 63c2 is fixed to an end portion 64e of a second surface portion 64b of the second lid part 64 with a screw 64s.

The control board 65a that controls lighting of the ultraviolet lamp units 31 is attached on the first lid part 63 inside the electric component box 61. When the first lid part 63 is moved to the position shown in Fig. 12, the electric component box 61 is opened and the inside of the electric component box 61 becomes visible. Specifically, the first lid part 63, which covers most of the electric component box 61, horizontally rotates approximately 90° around the first hinge portion h1. With the movement of the first lid 63, the power terminal block 65b and the external control terminal block 65c in the base part 62 that are covered with the first lid part 63, and the control board 65a attached to the first lid part 63 are exposed.

In the state where only the first lid part 63 is opened as shown in Fig. 12, it is possible to inspect the electric component 65, including the control board 65a, while continuing an operating state of the electric component 65 and checking the state of the ultraviolet lamp units 31 through the inspection window 52 of the second maintenance panel 51.

As shown in Fig. 13, the second lid part 64 is L-shaped. The second lid part 64 has a first surface portion 64a facing the second maintenance panel 51, and the second surface portion 64b that constitutes the front surface portion 61e of the electric component box 61. One end portion 64c of the first surface portion 64a is rotatably attached to the second hinge portion h2 of the base part 62. The other end portion 64d of the first surface portion 64a continues to the second surface portion 64b at a right angle. The first lid part is fixed to the end portion 64e of the second surface portion 64b is fixed to 63 with a screw 64s.

The second lid part 64 includes a bracket 66 that is attached to a region of the second surface portion 64b on the side of the blowout port 14 of the casing 12 of the sterilization unit 11 and that is fixed to the second maintenance panel 51 with a screw 51s.

When the second lid part 64 is moved to the position shown in Fig. 14, the second maintenance panel 51 is ready to be removed. Specifically, the second lid part 64 that blocks the second maintenance panel 51 horizontally rotates approximately 90° around the second hinge portion h2. With the movement of the second lid part 64, the second maintenance panel 51 that is covered with the second lid part 64 is exposed. The second maintenance panel 51 is attached to the casing 12 of the sterilization unit 11 with the screw 51s, and the second maintenance panel 51 can be removed from the casing 12 by unscrewing the screw 51s.

Next, the detection mechanism 71 that detects opening and closing of the second lid part 64 is described using Figs. 13 to 15.

The detection mechanism 71 of the sterilization unit 11 according to the present embodiment includes, as main elements, a microswitch 72 serving as a detection unit and a piece portion 73 serving as a detection target.

As shown in Fig. 15, the microswitch 72 that is the detection unit is attached at a position on the second surface portion 64b inside the electric component box 61, the position corresponding to the rear side of the bracket 66. The microswitch 72 is a switch unit that supplies and stops power to the electric component 65. The microswitch 72 has a lever 72a that supplies power to the electric component 65 when being pressed and that returns to a return position and stops power supply when pressing force is lost. The second maintenance panel 51 includes the piece portion 73 that presses the lever 72a of the microswitch 72. The first surface portion 64a of the second lid part 64 has a slit 64f disposed in the vicinity of the bracket 66 to allow the piece portion 73 to pass through.

As shown in Fig. 13, while the second lid part 64 is closed, and specifically, while the second lid part 64 blocks the second maintenance panel 51, i.e., while the second lid part 64 is in a prescribed position, the piece portion 73 is inserted into the electric component box 61 through the slit 64f and presses the lever 72a of the microswitch 72. When the lever 72a is pressed, the microswitch 72 is put in an ON state, so that power is supplied to the electric component 65 including the control board 65a.

On the other hand, as shown in Fig. 14, while the second lid part 64 is opened, and specifically, while the second lid part 64 does not block the second maintenance panel 51, i.e., while the second lid part 64 is not in the prescribed position, the piece portion 73 is released through the slit 64f and is separated from the lever 72a of the microswitch 72. When the force to press the lever 72a is released, the microswitch 72 is put in an OFF state, so that power supply to the electric component 65 including the control board 65a is stopped.

During the inspecting work and the replacing work of the ultraviolet lamp units 31, it is necessary to move the second lid part 64 to the position shown in Fig. 13 and then remove the second maintenance panel 51 to put the access hole 17b for the ultraviolet lamp units 31 in an open state as shown in Fig. 14. In the state shown in Fig. 13, the detection mechanism 71 has already stopped power supply to the control board 65a and therefore the ultraviolet lamps 32 are turned off. This ensures that the ultraviolet lamps 32 are turned off when the second maintenance panel 51 is removed. Therefore, there is no risk that an operator directly looks at ultraviolet light.

Even if the second lid part 64 should be closed without attaching the second maintenance panel 51 at the end of the inspecting work and the replacing work of the ultraviolet lamp units 31, the detection mechanism 71 maintains the OFF state that is the state where power supply is stopped, so that the ultraviolet lamps 32 are not lit. This ensures the safety of the operator.

As described above, power supply to the ultraviolet lamp unit 31 is cut off by opening the second lid part 64 of the electric component box 61 or removing the electric component box 61 from the casing 12. Consequently, even when the operator accidentally attempts to perform inspection operation of the sterilization unit 11 while the ultraviolet lamp unit 31 is turned on, opening the second lid part 64 or removing the electric component box 61 from the casing 12 eliminates the risk of the operator directly looking at the ultraviolet light.

However, when the panel fixing screws 17s of the first maintenance panel 41 and the second maintenance panel 51 are removed before the second lid part 64 is opened or the electric component box 61 is removed from the casing 12, there is the risk of the ultraviolet light leaking through the screw fixing holes 401s to 406s, the screw fixing holes 501s to 507s, the screw holes 401 to 406, and the screw holes 501 to 507.

In the present embodiment, the plurality of screw holes 401 to 406 and 501 to 507 of the first side plate 17 include three screw holes 405, 406, and 501 that have the risk of leaking the ultraviolet light and a plurality of screw holes that have no risk of leaking the ultraviolet light.

For example, the shading grille 21 is provided between the screw holes 401, 402, and 507 and the ultraviolet lamp unit 31. Accordingly, the ultraviolet light emitted by the ultraviolet lamp unit 31 is blocked by the shading grille 21 and does not reach the screw holes 401, 402, and 507. In other words, there is no risk that the ultraviolet light leaks through the screw holes 401, 402, and 507. The other screw holes 403, 404, and 502 to 506 are hidden by a portion where the electric component box 61 is attached. It is not possible to remove the panel fixing screws 17s from these screw holes 403, 404, and 502 to 506 without removing the electric component box 61 first. In other words, there is no risk that the ultraviolet light leaks through the screw holes 403, 404, and 502 to 506.

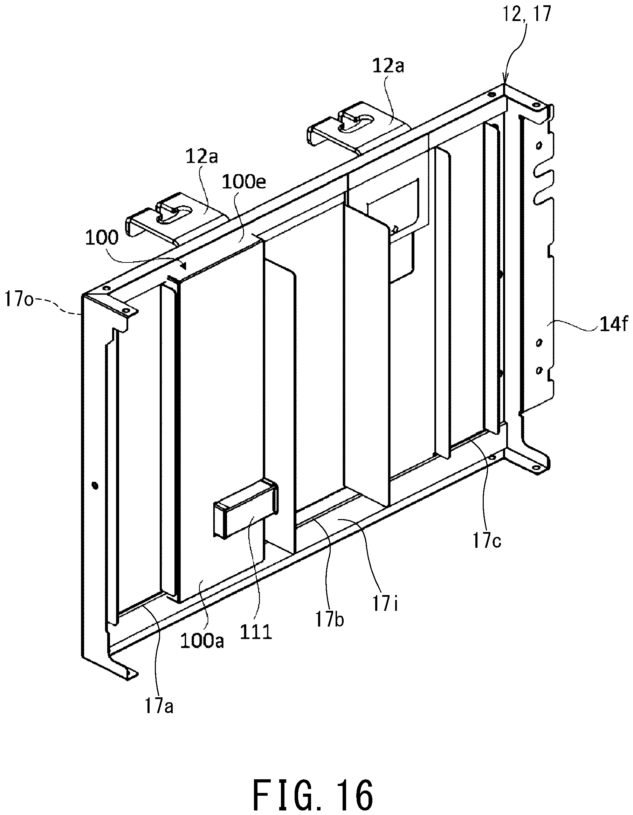

Fig. 16 is a perspective view of the first side plate 17 as viewed from the inside of the casing 12.

As shown in Fig. 16, the casing 12 includes an internal casing 100 provided on an inner side surface 17i of the first side plate 17, and a cable cover 111 combined with the internal casing 100. The internal casing 100 is fixed to a part of the inner side surface 17i of the first side plate 17 that is between the access hole 17a of the shading grille 21 on the side of the suction port 13 and the access hole 17b of the ultraviolet lamp unit 31. The height of the internal casing 100 is approximately the same as the first side plate 17. In the present embodiment, the internal casing 100 is provided so that screw holes to be shielded described later are all hidden as viewed from the inner side of the casing 12.

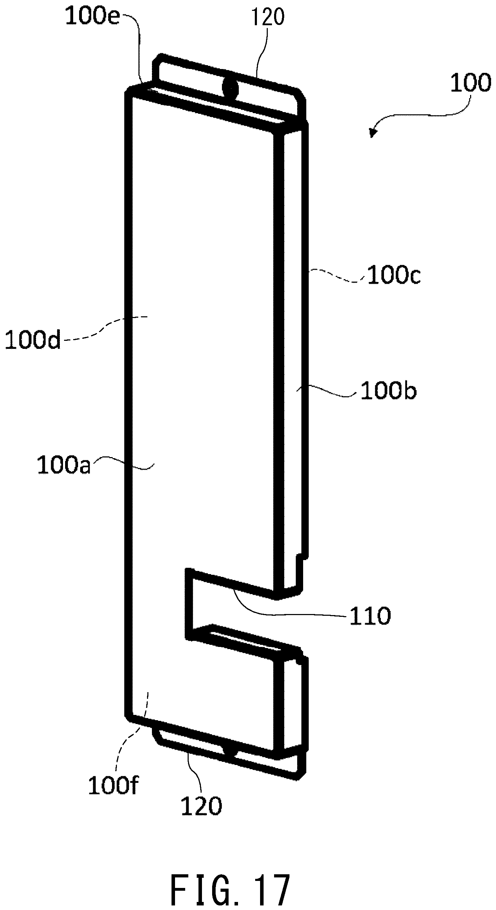

Fig. 17 is a perspective view of the internal casing 100.

As shown in Fig. 17, the internal casing 100 has an approximately rectangular box shape. For details, the internal casing 100 is a box body formed by bending a metal plate such as a single sheet metal. The internal casing 100 has a front surface plate 100a, a left side surface plate 100b, a right side surface plate 100d, a top surface plate 100e, and a bottom surface plate 100f. A rear surface 100c of the internal casing 100 is open. The height of the internal casing 100 is approximately the same as the first side plate 17.

The top surface plate 100e and the bottom surface plate 100f of the internal casing 100 each have a fixed piece portion 120 that extends perpendicularly to the surface and extends in the longitudinal direction of the casing 12. The respective fixed piece portions 120 are disposed on the rear surface 100c side of the top surface plate 100e and the bottom surface plate 100f.

The front surface plate 100a and the left side surface plate 100b further have an opening portion 110 in a partially notched shape. The cable cover 111 is provided in the internal casing 100 so as to close the opening portion 110.

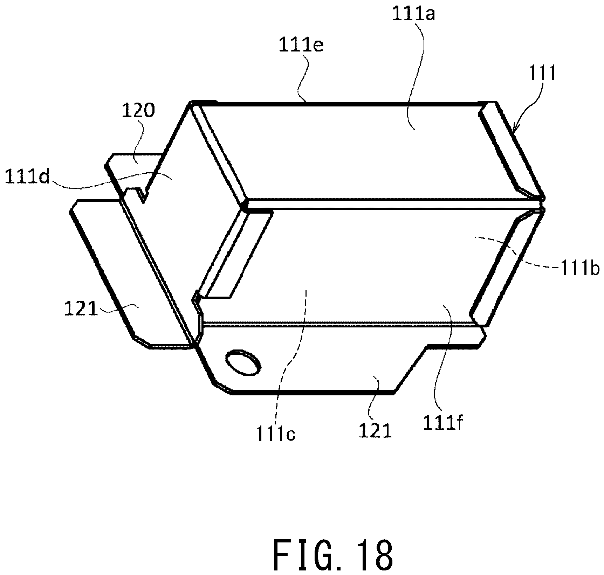

Fig. 18 is a perspective view of the cable cover 111.

As shown in Fig. 18, the cable cover 111 has an approximately rectangular box shape. The cable cover 111 is a box body formed by bending a metal plate such as a single sheet metal mostly in the same manner as the internal casing 100. The cable cover 111 has a front surface plate 111a, a right side surface plate 111d, a top surface plate 111e, and a bottom surface plate 111f. A left side surface 111b and a rear surface 111c of the cable cover 111 are open.

The top surface plate 111e, the bottom surface plate 111f, and the right side surface plate 111d each have a fixed piece portion 121 that extends perpendicularly to the surface. The respective fixed piece portions 121 are disposed on the rear surface 111c side of the top surface plate 111e, the bottom surface plate 111f, and the right side surface plate 111d.

The fixed piece portions 121 of the cable cover 111 are spot welded at prescribed positions on the inner side of the first side plate 17. The internal casing 100 is installed so that the cable cover 111 and the opening portion 110 are fitted to each other. The fixed piece portions 120 of the internal casing 100 are spot welded to the inner side surface 17i of the first side plate 17.

Thus, the internal casing 100 and the cable cover 111 are so-called a sheet metal workpiece made by folding a single metal plate. Accordingly, the internal casing 100 and cable cover 111 are free from the risk of weld failure that occurs when plates are welded together. Consequently, the internal casing 100 and the cable cover 111 are extremely unlikely to leak the ultraviolet light emitted by the ultraviolet lamp unit 31.

Furthermore, there is no need for threading in both the process of working the internal casing 100 and the cable cover 111 and the process of fixing the internal casing 100 and the cable cover 111 to the first side plate 17. As a result, the number of component parts of the casing 12 is reduced, and ultraviolet light does not leak through the screw holes.

Furthermore, as shown in Fig. 16, double structure is adopted in which about the half of the respective surfaces of the top surface plate 111e, the bottom surface plate 111f, and the right side surface plate 111d of the cable cover 111 is covered with the internal casing 100. Consequently, the internal casing 100 and the cable cover 111 can prevent ultraviolet light from leaking through the opening portion 110 of the internal casing 100.

When the internal casing 100 and the cable cover 111 are fixed to the inner side surface 17i of the first side plate 17, the front surface plate 100a of the internal casing 100 having the opening portion 110 is blocked by the cable cover 111, and the left side surface plate 100b having the opening portion 110 is in a line with the left side surface 111b of the cable cover 111. In other words, the internal casing 100 and the cable cover 111 are open only at the left side surface 111b of the cable cover 111 as viewed from the inner side of the casing 12.

As shown in Figs. 11 and 16, an outer side surface 17o of the first side plate 17 has an opening portion 17e that matches with the rear surface 111c of the cable cover 111. Consequently, the inside and outside of the casing 12 are connected through the opening portion 17e of the first side plate 17, the opening of the rear surface 111c of the cable cover 111, the inner side of the cable cover 111, and the opening of the left side surface 111b of the cable cover 111. This route is an approximately rectangular wiring passage. The opening portion 17e is also connected to the outside of the casing 12 without being closed by the base part 62 and the second lid part 64 of the electric component box 61.

The wiring passage houses a cable 300 for supplying power source to the ultraviolet lamp unit 31 as shown in Fig. 12.

When the first maintenance panel 41, the second maintenance panel 51, and the electric component box 61 are attached to the casing 12, the opening portion 17e of the first side plate 17 matches with a wiring hole 62b provided on the base part 62 of the electric component box 61 as shown in Fig. 12.

The cable 300 connected to the ultraviolet lamp unit 31 in the casing 12 is routed through the wiring passage and wired into the electric component box 61 through the wiring hole 62b. The cable 300 is also connected to the control board 65a in the electric component box 61. The wiring hole 62b is preferably provided with a measure to prevent leakage of ultraviolet light so that there is no risk that the ultraviolet light leaks through the wiring hole 62b.

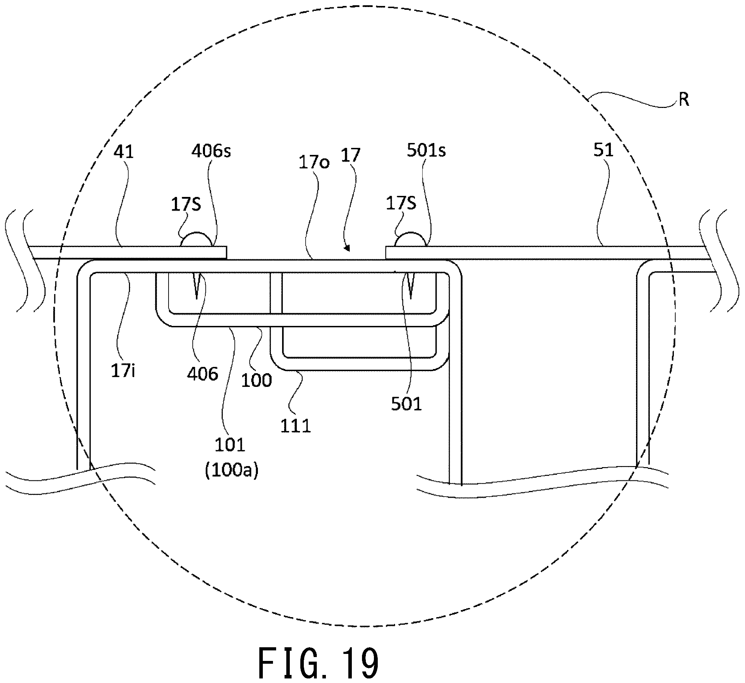

Fig. 19 is an enlarged view of an R portion of Fig. 4, when the first maintenance panel 41 and the second maintenance panel 51 are attached to the first side plate 17.

As shown in Fig. 19, the casing 12 includes a shielding portion 101 that is provided on the inner side surface 17i of the first side plate 17 that includes the screw holes 405 (not shown), 406, and 501 having the risk of leaking ultraviolet light, the shielding portion 101 facing the first side plate 17. The shielding portion 101 of the present embodiment is the front surface plate 100a of the internal casing 100. Here, the screw holes 405, 406, and 501, which are required to face the shielding portion 101 as they have the risk of leaking ultraviolet light, are simply referred to as the "screw holes to be shielded" below.

The shielding portion 101 is installed so as to face the screw holes to be shielded at a prescribed interval from the screw holes to be shielded. The interval is a distance long enough to prevent the panel fixing screws 17s, which are screwed into the screw holes to be shielded, from penetrating the shielding portion 101, and more preferably a distance long enough to prevent the panel fixing screws 17s from touching the shielding portion 101.

Specifically, the casing 12 includes the shielding portion 101 that faces the screw holes to be shielded of the first side plate 17 and that does not include any route, such as a hole, that allows the ultraviolet light to pass. This prevents the ultraviolet light emitted by the ultraviolet lamp unit 31 from traveling straight through the screw holes to be shielded and leaking to the outside of the casing 12.

The front surface plate 100a of the internal casing 100 in the present embodiment has the opening portion 110, though the opening portion 110 is blocked by the cable cover 111. Consequently, there is no risk that the ultraviolet light leaks from the opening portion 110.

Thus, the sterilization unit 11 according to the present embodiment includes the front surface plate 100a of the internal casing 100 as the shielding portion 101 provided inside the screw holes to be shielded of the first side plate 17 of the casing 12. Consequently, even when the panel fixing screws 17s for fixing the first maintenance panel 41 and the second maintenance panel 51 are removed before the electric component box 61 is detached, there is no risk that the ultraviolet light leaks through the screw holes to be shielded of the first side plate 17.

The shielding portion 101 is provided as part of the approximately rectangular shaped internal casing 100. As a result, it is possible to prevent leakage of the ultraviolet light more reliably than the case of providing only the shielding portion 101 as an independent component.

The shielding portion 101 may include, in place of the front surface plate 100a of the internal casing 100, a member having at least a surface that functions as the shielding portion 101 that is approximately the same size as the screw holes to be shielded, the member facing the inner side surface 17i of the first side plate 17. In other words, the shapes of the shielding portion 101 and the internal casing 100 can be changed as necessary within the range that the ultraviolet light emitted toward the screw holes to be shielded can be blocked.

In addition, an installation location of the shielding portion 101 is not limited to the screw holes to be shielded, and the shielding portion 101 can be applied to portions having the risk of leaking the ultraviolet light. For example, the shielding portion 101 can be installed on the first side plate 17 as well as various portions, such as another side surface plate and the bottom surface plate. The shielding portion 101 may also be provided in portions other than the screw holes when the portions have the risk of leaking the ultraviolet light.

Furthermore, the shapes of the rear surface 100c and the opening portion 110 of the internal casing 100 having the shielding portion 101 are not limited to the rectangular shape. For example, when it is possible to substitute the left side surface plate 100b or the right side surface plate 100d of the internal casing 100 with some portions of the first side plate 17, such as a flange of an opening edge of the access hole 17a of the shading grille 21 that is disposed on the suction port 13 side of the casing 12 or a flange provided at the access hole 17b of the ultraviolet lamp units 31, the internal casing 100 may not include the left side surface plate 100b or the right side surface plate 100d. The cable cover 111 may be provided at a distance from the internal casing 100. In other words, the cable cover 111 and the internal casing 100 may be provided independently.

It is also possible to separately provide the shielding portions 101, which are provided at least to have approximately the same size as the screw holes to be shielded. Specifically, since there are three screw holes to be shielded in the present embodiment, the internal casing 100 that functions as the shielding portion 101 may be provided for each of the three screw holes, or the three screw holes may be divided into two screw holes and one screw hole, so that two shielding portions 101 may be provided for the two screw holes and the one screw hole, respectively.

Although there are three screw holes to be shielded, the number of the screw holes to be shielded is not limited to three, and the present invention is applicable to the cases where there are a plurality of screw holes having the risk of leaking the ultraviolet light.

Furthermore, the internal casing 100 may have a hollow inside or contain a heat insulating member or the like.

Although some of the embodiments of the present invention have been described in the foregoing, the embodiments are merely illustrative and are not intended to restrict the scope of the invention. These new embodiments can be performed in other various forms, and various kinds of omissions, replacements and modifications are possible without departing from the meaning of the invention. These embodiments and their changes are not only embraced in the range and scope of the invention but are also embraced in the invention set forth in the claims and in the range of the equivalency thereof.

For example, in the present embodiment, the sterilization unit 11 is directly connected to the duct-type air conditioning indoor unit 1, though the air conditioning indoor unit 1 and the sterilization unit 11 may be connected indirectly. For example, a relay duct may be provided between the air conditioning indoor unit 1 and the sterilization unit 11. In place of the duct-type air conditioning indoor unit 1, an outside air processing indoor unit that incorporates a total heat exchanger may be connected to the sterilization unit.

Moreover, the sterilization unit 11 may include a high-performance filter in place of the shading grilles 21 disposed on the side of the blowout port 14. In this case, the high-performance filter captures remaining germs that have passed through the ultraviolet lamp units 31, and the ultraviolet lamps 32 can irradiate the high-performance filter with ultraviolet light to eliminate the germs captured by the high-performance filter. As a result, the sterilization performance of the sterilization unit 11 is further enhanced.

Furthermore, the detection mechanism 71 may include an optical sensor or a magnetic sensor capable of detecting whether the second lid part 64 blocks the second maintenance panel 51, in place of the microswitch 72 and the piece portion 73.

### Reference Signs List

1...indoor unit, 2...casing, 3...blower, 4...heat exchanger, 5...drain pan, 6...suction port, 7...blowout port, 11...sterilization unit, 12...casing, 12a...hanging metal fitting, 12b...column, 13...suction port, 14...blowout port, 14f...connection flange, 15...top plate, 16...bottom plate, 17...first side plate, 17a...access hole, 17b...access hole, 17c...access hole, 17d...inspection hole, 18...second side plate, 19a...guide rail, 19b...guide rail, 19c...guide rail, 21...shading grille, 22...louver, 22a...first plate portion, 22b...second plate portion, 221...apex, 222, 223...lower end, 224...virtual line, 23... frame body, 230...L-shaped member, 231...longitudinal member, 232...transverse member, 234... engagement portions, 25...protrusion, 250...seal member, 26...coupling mechanism, 261...coupling hook portion, 261a...hook groove, 262...coupling projection portion, 31...ultraviolet lamp unit, 32...ultraviolet lamps, 33...frame body, 41...first maintenance panel, 51...second maintenance panel, 51s...screw, 52...inspection window, box 61...electric component, 61a...upper surface portion, 61b...lower surface portion, 61c...outer side surface portion, 61d...inner side surface portion, 61e...front surface portion, 61f...rear surface portion, 62...base part, 62a...fixed portion, 63...first lid part, 63c1...first corner portion, 63c2... second corner portion, 64...second lid part, 64a...first surface portion, 64b...second surface portion, 64c, 64d, 64e...end portion, 64f...slit, 64s...screw, 65...electric component, 65a...control board, 65b...power terminal block, 65c...external control terminal block, 66...bracket, 71... detection mechanism, 72...microswitch, 72a...lever, 73...piece portion, S1...indoor air suction port, S2...air-conditioned air blowout port, S3...maintenance hatch, P1...suction duct, P2...blowout duct, h1...first hinge portion, h2... second hinge portion.

## Claims

1. A sterilization unit, comprising:
a casing;
an ultraviolet lamp that sterilizes air passing through the casing;
a control board for the ultraviolet lamp; and
an electric component box that houses the control board,
wherein the casing includes
an internal casing provided inside the casing, and
a shielding portion that is part of the internal casing to prevent ultraviolet light emitted by the ultraviolet lamp from leaking to outside of the casing.

2. The sterilization unit according to claim 1, comprising a maintenance panel that is detachably attached to an outer surface of the casing,
Wherein the shielding portion prevents the ultraviolet light from leaking to the outside of the casing through screw holes for attaching the maintenance panel.

3. A sterilization unit, comprising:
a casing;
a maintenance panel that is detachably attached to an outer surface of the casing;
an ultraviolet lamp that sterilizes air passing through the casing;
a control board for the ultraviolet lamp; and
an electric component box that houses the control board,
wherein the casing includes a shielding portion that prevents ultraviolet light emitted by the ultraviolet lamp from leaking to outside of the casing through screw holes for attaching the maintenance panel.

4. The sterilization unit according to claim 3, wherein
the shielding portion is part of an internal casing provided inside the casing.

5. The sterilization unit according to claim 1 or 4, wherein
the internal casing covers part of a cable cover that houses a cable connected to the ultraviolet lamp.
